(19) Europäisches Patentamt
European Patent Office
Office européen des brevets

(11) Veröffentlichungsnummer : **0 135 776**
**B1**

# (12) EUROPÄISCHE PATENTSCHRIFT

(45) Veröffentlichungstag der Patentschrift : **11.10.89**

(51) Int. Cl.$^4$ : **C 07 C 51/353**, C 07 C 53/08, B 01 J 31/18

(21) Anmeldenummer : **84109732.2**

(22) Anmeldetag : **16.08.84**

## (54) Katalysatorsystem und Verfahren zur Herstellung von Essigsäure durch katalytische Umlagerung von Ameisensäuremethylester.

(30) Priorität : **15.09.83 DE 3333317**

(43) Veröffentlichungstag der Anmeldung : **03.04.85 Patentblatt 85/14**

(45) Bekanntmachung des Hinweises auf die Patenterteilung : **11.10.89 Patentblatt 89/41**

(84) Benannte Vertragsstaaten : **BE DE FR GB IT NL**

(56) Entgegenhaltungen :
**EP--A-- 0 055 622**
**EP--A-- 0 105 132**
**EP--A-- 0 109 212**
**FR--A-- 2 303 597**

(73) Patentinhaber : **HÜLS AKTIENGESELLSCHAFT**
**- RSP Patente / PB 15 - Postfach 13 20**
**D-4370 Marl 1 (DE)**

(72) Erfinder : **Hög, Hans-Ulrich, Dr.**
**Bitterfelder Strasse 9a**
**D-4370 Marl (DE)**
Erfinder : **Bub, Günther, Dr.**
**Kampstrasse 94**
**D-4370 Marl (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 135 776 B1

Jouve, 18, rue St-Denis, 75001 Paris, France

## Beschreibung

Die Erfindung betrifft ein Verfahren zur Herstellung von Essigsäure durch Umlagerung von Ameisensäuremethylester. Das erfindungsgemäß eingesetzte Katalysatorsystem enthält Rhodium bzw. Salze oder Komplexe desselben, als Promotor ein Halogen oder eine Halogenverbindung und Liganden aus der Gruppe der Carbonsäureamide, gegebenenfalls zusätzlich als eine zweite Metallkomponente ein Salz oder einen Komplex eines Elementes der VI. Nebengruppe. Die Umlagerung wird in Gegenwart von Kohlenmonoxid in einem praktisch wasserfreien Medium durchgeführt.

Die katalytische Umlagerung von Ameisensäuremethylester zu Essigsäure mit carbonylbildenden nichtedlen Metallen der VI. bis VIII. Gruppe oder deren Verbindungen in Gegenwart von Promotoren, die Vanadium, Arsen, Antimon oder Wismut sowie Halogen enthalten, wird in der DE-PS 10 72 979, der GB-PS 628 161 und der US-PS 2 508 513 beschrieben; näher ausgeführt werden Katalysatorsysteme, die Nickel oder Cobalt sowie Jod und Wismut erhalten. Diese Verfahren benötigen drastische Reaktionsbedingungen wie Temperaturen von mehr als 200 bis 335 °C und Drücke von etwa 300 bar; die Essigsäureselektivitäten sind unbefriedigend, und selbst nach langer Umsetzungsdauer wird häufig nur ein mäßiger Umsatz erreicht.

Ähnlich scharfe Bedingungen werden bei Katalysatorsystemen angewendet, in den Cobalt, Quecksilber, Eisen, Nickel oder Zink als katalytisch aktive Metalle gemeinsam mit Brom- oder Jodpromotoren und stickstoffhaltigen Lösemitteln eingesetzt werden, die in der DE-PS 20 26 031 (= US-PS 3 839 428) und in der JP-AS 16 733/75 beschrieben sind. Zwar werden dabei bessere Selektivitäten für Essigsäure erreicht, jedoch sind die spezifischen Katalysatorleistungen, ausgedrückt als Gramm Essigsäure pro Gramm Metall und Stunde, nur mäßig. Die DE-PS 20 26 031 beansprucht die Metalle der Gruppen VIII oder II b, darunter auch Rhodium, als katalytisch aktive Komponenten.

Im Beispiel 22 dieser DE-PS bzw. Example 26 der äquivalenten US-PS wird ein Katalysatorsystem aus Rhodiumchlorid, N-Methylpyrrolidon und Jodwasserstoff beschrieben. Die Konzentrationen an Rhodium- (150 mg-at Rh/mol Methylformiat) Jod-(240 mg-at J/mol Methylformiat) und Stickstoffverbindung (3,6 mol/mol Methylformiat) sind außerordentlich hoch, die Katalysatorleistung mit weniger als 1 g Essigsäure pro Gramm Rhodium und Stunde gering. Gemäß den Ansprüchen dieser DE-PS benötigt man die organische Stickstoffverbindung als Lösemittel in einer Menge von mindestens 0,2 mol pro mol Methylformiat. Der Reaktionsdruck muß insgesamt mindestens 80 bar, der Partialdruck des Kohlenmonoxids mindestens 50 bar betragen.

Die ältere, aber nicht vorveröffentlichte EP-PS 109 212 offenbart ein Verfahren zur Herstellung einer Carbonsäure durch Umlagerung eines Esters der Ameisensäure in Gegenwart von Kohlenmonoxid und eines Katalysatorsystems, das Rhodium, ein Halogenid und eine Verbindung mit einem quaternären Atom der Gruppe V des Periodensystems der Elemente enthält. Hierbei kann auch diese Verbindung mit einem dreiwertigen Atom der Gruppe V eingesetzt und mit Hilfe eines Quaternisierungsmittels unter den Reaktionsbedingungen quaternisiert werden.

Mit Nickel als Katalysatormetall können gemäß der JP-OS 56-73040 bei technisch interessanten Bedingungen wie 180 °C und 50 bar Reaktionsdruck hohe Ausbeuten erzielt werden. Die Umsetzung erfolgt in Gegenwart einer organischen Verbindung eines Elements der V. Hauptgruppe und einer großen Menge einer Jodverbindung.

Nachteilig ist, daß hohe Ausbeuten von 90 % und mehr nur bei gleichzeitigem Zusatz eines den Katalysator und das Edukt stabilisierenden Lösemittels und von Methylacetat erreicht werden. Nicht nur die spezifische Katalysatorleistung, die nur weniger als 15 g Essigsäure pro Gramm Nickel und Stunde erreicht, ist mäßig, sondern durch das Verdünnen mit einem Lösemittel wird auch die Raumzeitausbeute vermindert.

Die Verwendung von Rhenium- oder Rutheniumträgerkatalysatoren in einer Gasphasenreaktion, wie in der JP-OS 65 703/73 beschrieben, oder eines Palladiumkatalysators, einer Jodverbindung und einer tertiären stickstoff- oder phosphororganischen Verbindung in der Flüssigphase gemäß der JP-OS 56-22745 führt zu deutlich schlechteren Ergebnissen als im Fall des vorstehenden Nickelkatalysatorsystems.

Im Falle der JP-OS 56-22745 finden sich zudem große Teile des umgesetzten Eduktes nicht im Zielprodukt Essigsäure oder dem zu dieser umwandelbaren Methylacetat wieder. Gemäß DE-OS 30 46 899 werden mit einem Katalysator, bestehend aus einer Palladium-, deutlich weniger gut auch Iridium- oder Ruthenium-, und einer Jodverbindung sowie einer organischen Verbindung der V. Hauptgruppe in Essigsäure oder Methylacetat als Lösemittel unter gleichartigen Reaktionsbedingungen ähnlich gute Ausbeuten an Essigsäure erreicht wie mit dem oben genannten Nickelkatalysator. Die gleichen Nachteile wie dort treffen auch hier zu, wobei der teure Palladiumkatalysator eine nur geringfügig höhere spezifische Leistung zeigt.

Mit Rhodiumkatalysatoren läßt sich eine bedeutend höhere Katalysatorleistung erzielen. Nach dem Stand der Technik, wie er in der DE-PS 21 09 025 (= US-PS 3 798 267) und der US-PS 4 194 056 offenbart ist, ist jedoch außer einem Jodpromotor auch der Zusatz eines Stiban-, Arsan- oder vorzugsweise Phosphanliganden notwendig, um bei der Durchführung der Reaktion unter vergleichsweise milden Bedingungen wie nicht mehr als 200 °C und nicht mehr als 50 bar Kaltdruck die Stabilität des katalytisch aktiven Komplexes und eine hohe Essigsäureselektivität zu gewährleisten. Zudem sind sehr hohe

Halogen/Rhodiumverhältnisse erforderlich, um eine hohe spezifische Katalysatorleistung zu erreichen. In einem Beispiel der US-PS 4 194 056 wird mit 0,84 mg-atom Rh/mol Methylformiat in Form von Rhodiumtrichlorid und Methyljodid als Promotor in dem Verhältnis J/Rh = 18,8 bei 200 °C und einem Kohlenmonoxidkaltdruck von 34 bar, der zu einem Reaktionsdruck von 50 bar führt, ohne Einsatz von Phosphan in zweistündiger Reaktion ein flüssiger Austrag erhalten, der noch zu 65 Gewichtsprozent aus unumgesetztem Edukt besteht und nur 24 Gewichtsprozent Essigsäure enthält.

Die spezifische Katalysatorleistung kann daraus zu höchstens 62 g Essigsäure pro Gramm Rhodium und Stunde abgeschätzt werden. In einem Beispiel der DE-PS 21 09 025 wird mit dem gleichen Katalysator, jedoch mit 0,31 mg-atom Rh/mol Methylformiat und einem Atomverhältnis J/Rh = 258 bei 200 °C und einem Kohlenmonoxidkaltdruck von 50 bar, der zu einem Reaktionsdruck von etwa 75 bar führt, in dreistündiger Reaktion ein Umsatz des Methylformiats von 99 % bei einer Essigsäureausbeute von 80,4 % erzielt, was einer Menge von 497 g Essigsäure pro Gramm Rhodium und Stunde entspricht.

Nach einem weiteren Beispiel derselben Patentschrift erhält man mit dem Rh-(I)-Komplex $[Rh(CO)_2Cl]_2$ als Rhodiumquelle unter sonst identischen Bedingungen sogar nur einen Umsatz von 43,2 %, eine Essigsäureausbeute von 29,6 % und damit eine Katalysatorleistung von 57 g Essigsäure pro Gramm Rhodium und Stunde.

Die unbefriedigende Wirkung von Rhodiumkatalysatorsystemen ohne Phosphanliganden geht aus den zitierten Beispielen deutlich hervor. Andererseits können solche Liganden Veränderungen unterliegen, die zur Desaktivierung des Katalysatorsystems führen. Zum Beispiel können die meist oxidationsempfindlichen Phosphane durch Spuren von Sauerstoff angegriffen werden. Weiterhin ist z. B. aus den US-PSS 4 260 828 und 4 283 304 bekannt, daß Rhodium-Katalysatoren unter ganz ähnlichen Druck- und Temperaturverhältnissen die langsame Zersetzung von Phosphanen bewirken. Die Bildung phosphidoverbrückter Rhodiumcluster vermindert die Wirksamkeit des Katalysators.

Um die zu erwartende Verlangsamung der Umsetzung zu vermeiden, müßten Maßnahmen wie Erhöhung der Reaktionstemperatur, der Katalysatormenge oder des Promotor/Metall-Verhältnisses ergriffen oder eine Aufarbeitung des Katalysators eingeschaltet werden, was zusätzliche Kosten verursachen würde.

Die bisher bekannten Verfahren zur übergangsmetallkatalysierten Umlagerung von Methylformiat weisen demnach Nachteile auf wie das Erfordernis scharfer Reaktionsbedingungen oder unwirtschaftlich großer Mengen an Katalysatorkomponenten, die Notwendigkeit von Lösemitteln oder empfindlichen Liganden, bzw. sie besitzen eine geringe Aktivität oder Selektivität.

Bei Verfahren zur Carbonylierung von Alkoholen zu Carbonsäuren, z. B. von Methanol zu Essigsäure, wird in der DE-PS 17 67 151 (= US-PS 3 689 533) die Anwendung eines Rhodium-Halogen-Katalysators mit Aminliganden beschrieben. Sofern als Edukt Ester des Alkohols eingesetzt werden, muß man aber in Gegenwart von Wasser arbeiten, um den Alkohol freizusetzen ; dabei beginnt die Reihe der Ester bei denen der Essigsäure.

Die Verwendung einer Chromkomponente in der DE-AS 19 66 695 (= US-PS 3 717 670) bezieht sich auf ein Gasphasenverfahren mit vergleichsweise geringer Katalysatoraktivität, wobei mit Estern als Edukt gleichfalls Wasser benötigt wird.

In der noch unveröffentlichten DE-Anmeldung 32 36 351 ist ein hochwirksames Katalysatorsystem für die Umlagerung von Methylformiat, das aus einer Rhodiumkomponente, die keine Liganden der V. Nebengruppe enthält, einer Verbindung eines Elementes der VI. Nebengruppe und einem Halogenpromotor besteht, beschrieben. Die Verwendung einer Chromverbindung ist auch für das Nickelkatalysatorsystem der JP-OS 56-73040 beschrieben, führt dort jedoch nur zu einer Essigsäureausbeute von 73,7 % und läßt keinen Vorteil gegenüber dem chromfreien Katalysator erkennen. Für das Verfahren der DE-OS 30 46 899, dessen grundsätzliche Nachteile schon aufgezeigt wurden, werden auch Verbindungen der Metalle der VI. Nebengruppe als « weniger bevorzugte sekundäre Promotoren » an Stelle der oder gemeinsam mit den bevorzugten organischen Verbindungen der Elemente der V. Hauptgruppe genannt ; ihre Wirkung wird jedoch nicht offenbart.

Es stellte sich daher die Aufgabe, ein Katalysatorsystem hoher Aktivität für die Umlagerung von Ameisensäuremethylester zu Essigsäure zu finden, mit dem Essigsäure in hoher Selektivität ohne die geschilderten Nachteile hergestellt werden kann. Vor allem zu vermeiden waren dabei selektivitätsmindernde Einflüsse, die ein Entstehen größerer Mengen an Ameisensäure bewirken, da diese die Aufarbeitung des Produktes außerordentlich erschweren würden. Mit der Bezeichnung « Umlagerung » ist keine Aussage hinsichtlich des tatsächlichen Reaktionsablaufes beabsichtigt und somit keine Einschränkung des erfindungsgemäßen Verfahrens verbunden.

Die gestellte Aufgabe wird erfindungsgemäß entsprechend den Angaben der Patentansprüche gelöst. Dazu bringt man Ameisensäuremethylester hinreichend lange, im allgemeinen 0,1 bis 10 Stunden, bei einer erhöhten Temperatur von 140 bis 300 °C in Gegenwart von Kohlenmonoxid bei einem CO-Partialdruck bei Reaktionstemperatur von 2 bis 250 bar mit einem Katalysatorsystem in Kontakt, welches Rhodium, ein Rhodiumsalz oder einen Rhodiumkomplex, ein Halogen als Promotor und Liganden aus der Gruppe der Carbonsäureamide enthält, wobei jede der notwendigen Komponenten für sich oder in Form einer Verbindung mit einer der anderen Komponenten eingesetzt werden kann. Das Katalysatorsystem kann zusätzlich eine zweite Metallkomponente in Form einer Verbindung eines Elementes der VI. Nebengruppe enthalten. Das Katalysatorsystem bewirkt die Umlagerung von Methylformiat zu Essigsäure

in praktisch wasserfreiem Medium unter vergleichsweise milden Bedingungen auch ohne den Zusatz eines Lösemittels in ausgezeichneter Selektivität und bei hoher spezifischer Katalysatorleistung in überraschender und nicht vorhersehbarer Weise. Vor allem wird das Carbonsäureamid nicht als Lösemittel, sondern nur in geringer katalytischer Menge bezüglich des Edukts benötigt.

Rhodium kann elementar in fein verteilter Form, als anorganisches oder organisches Salz oder als Komplexverbindung eingesetzt werden. Dabei ist es vorteilhaft, daß sich aus so einfachen Verbindungen wie $RhCl_3 \cdot 3\ H_2O$, die eine ausgezeichnete Wirksamkeit für das Verfahren zeigen, unter Reaktionsbedingungen die aktive Katalysatorform ohne spezielle Präformierung schnell bildet. Weitere geeignete Einbringungsformen sind $Rh_2O_3$, $RhBr_3$, $RhJ_3$, Rhodium-(III)-acetylacetonat, Rhodium-(II)-acetat-Dimer, Chloro-dicarbonylrhodium-(I)-Dimer, Tetrarhodiumdodecacarbonyl und andere mehr. Geeignet sind auch Rhodiumkomplexe, die schon einen Carbonsäureamidliganden enthalten.

Als Halogenpromotoren eignen sich Jod und, mit schwächerer Promotorwirkung, Brom als Element oder in Form einer Verbindung. Geeignete Einsatzformen umfassen Verbindungen der Form $X_3^-$ und HX mit X = J oder Br, anorganische und organische Jodide und Bromide. Als anorganische Verbindungen können Salze derjenigen Metalle dienen, die die Reaktion ihrerseits nicht ungünstig beeinflussen, wie zum Beispiel Jodide und Bromide der Alkali- und Erdalkalimetalle oder von den Übergangsmetallen die des Nickels oder auch der Katalysatormetalle selbst, als organische Verbindungen Alkyl-, Acyl- und Aryljodide und -bromide. Diese Verbindungen können einzeln oder im Gemisch miteinander eingesetzt werden.

Geeignet sind auch Addukte einer organischen Jod- oder Bromverbindung mit dem Carbonsäureamidliganden. Um die erwünschten hohen Essigsäureausbeuten zu erhalten, sollte jedoch nicht die gesamte Menge an Promotor in dieser gebundenen, sondern ein Teil noch in freier Form vorliegen.

Entsprechend sollten Promotor und Carbonsäureamid in solchen Mengen eingesetzt werden, daß das Verhältnis von gesamter Menge an Jod- und Bromatomen zur gesamten Menge an Stickstoffatomen größer als 1 ist. Bevorzugt sind Jod und seine Verbindungen, insbesondere Methyljodid.

Geeignete Carbonsäureamide sind Form- oder Acetamid und ihre N-Alkyl- und N · N-Dialkylderivate sowie entsprechende Verbindungen höherer Carbonsäuren, zu denen beispielsweise auch aromatische Amide wie das Benzamid, N-Alkyl- und N · N-Dialkylbenzamide sowie Lactame wie Pyrrolidon, N-Alkylpyrrolidone, Piperidon und N-Alkylpiperidone gehören.

Bevorzugte Carbonsäureamide sind die Form- und vor allem die Acetamide, da mit ihnen dieselben Carbonsäurereste eingeführt werden, die schon zum Reaktionsgemisch gehören.

Bei den genannten Carbonsäureamiden ist keine eindeutige Abhängigkeit ihres Einflusses auf den Reaktionsablauf von der Basizität oder den sterischen Verhältnissen erkennbar, so daß dieser Einfluß im Einzelfall experimentell zu ermitteln ist, was nach der Lehre der vorliegenden Erfindung in einfacher Weise erfolgen kann. Tendenziell günstig scheinen jedoch mäßig basische Carbonsäureamide einerseits, solche mit kurzen Substituenten bis etwa $C_4$ am Stickstoff andererseits zu sein.

Ein wirksames Katalysatorsystem erhält man auch, wenn man als zweite Metallkomponente eine Verbindung eines Metalls der VI. Nebengruppe, bevorzugt eine Chrom- oder Molybdänverbindung, insbesondere eine Chromverbindung, zusetzt. Eine solche zweite Metallkomponente kann beispielsweise ein Carbonyl, Halogenid, Acetat, Acetylacetonat oder ein anderes organisches oder anorganisches Salz oder ein Komplex des Metalls sein.

Lösemittel zur Stabilisierung des Methylformiats sind nicht erforderlich, sondern wirken unter Umständen sogar schädlich. Der Zusatz von Essigsäure bringt jedoch keine Nachteile. Den Katalysator nicht beeinträchtigende Lösemittel oder Essigsäure selbst ermöglichen aber bei Durchführung der Umsetzung in der Flüssigphase die Anwendung niedrigerer Drücke, als sie sich sonst aufgrund des Eduktdampfdruckes einstellen würden. Die Umsetzung läuft in praktisch wasserfreiem Medium ab. Es ist vorteilhaft, praktisch wasserfrei zu arbeiten, da die Gegenwart von Wasser die Verseifung des Ameisensäureesters (Gl. 1) oder in Gegenwart des Produktes Essigsäure die Transesterifizierung (Gl. 2) fördert; dieser Effekt kann durch die thermische Zersetzung der gebildeten Ameisensäure (Gl. 3) verstärkt werden, und zu einem unerwünschten Druckanstieg im Reaktionsgefäß führen. Außerdem kann mit Wasser die Konvertierung von Kohlenmonoxid (Gl. 5) ablaufen, deren Katalyse durch Rhodiumverbindungen literaturbekannt ist.

$$HCOOCH_3 + H_2O \rightarrow HCOOH + CH_3OH \qquad (Gl. 1)$$

$$HCOOCH_3 + CH_3COOH \xrightarrow{[H_2O]} HCOOH + CH_3COOCH_3 \qquad (Gl. 2)$$

$$HCOOH \xrightarrow{\Delta T} H_2O + CO \qquad (Gl. 3)$$

$$HCOOH \xrightarrow{[Kat.]} H_2 + CO_2 \qquad (Gl. 4)$$

$$H_2O + CO \xrightarrow{[Kat.]} H_2 + CO_2 \qquad (Gl. 5)$$

Aus Methanol und Methylacetat ihrerseits können vor allem in Anwesenheit von Wasserstoff, im

Reaktor von vornherein vorhanden oder gemäß Gl. 4 gebildet, weitere Nebenprodukte entstehen, deren Gegenwart die Aufarbeitung erschwert. Auch Ameisensäure selbst im Produktgemisoh führt zu derartigen Schwierigkeiten. Wassermengen im Reaktionsgemisch von nicht mehr als 5 Gewichtsprozent, günstiger von nicht mehr als 2 Gewichtsprozent, können jedoch geduldet werden. Dabei ist es wegen des Herstellungsverfahrens für Ameisensäuremethylester unproblematisch, das Edukt praktisch wasserfrei zu erhalten.

Auch ein Gehalt von Methanol im Edukt, wie er für technisches Material üblich ist, stört nicht; Methanol wird ebenfalls in Essigsäure überführt. In Abwesenheit von Wasser ist außerdem die Korrosion des Reaktormaterials durch die produzierte Essigsäure und das Halogen des Promotors stark vermindert, so daß gegebenenfalls auf teure Sondermaterialien verzichtet werden kann. Weiter ist es vorteilhaft, daß das Produkt in praktisch wasserfreier Form anfällt.

Die Katalysatorkomponenten werden in der Flüssigphase suspendiert oder homogen gelöst, wobei die Metallkomponenten auch auf Trägern wie Aktivkohle, Aluminiumoxid oder Kieselgel aufgebracht sein können. Das Verfahren kann auch in der Gasphase durchgeführt werden, wobei die metallhaltigen Katalysatorbestandteile in fester Form oder auf einem Träger wie Aktivkohle, Kieselgel oder Aluminiumoxid aufgebracht mit einem gasförmigen Einsatzgemisch aus dem Edukt, einer flüchtigen Jod- oder Bromverbindung, einem Carbonsäureamid und Kohlenmonoxid in Kontakt gebracht werden. Bei hinreichender Schwerflüchtigkeit bringt man das Carbonsäureamid ebenfalls auf den Träger auf bzw. löst oder suspendiert die metallhaltigen Komponenten in der Schmelze. Bevorzugt ist aber die Durchführung in der Flüssigphase, die bessere Umsätze und/oder Selektivitäten gibt. Das Verfahren läßt sich kontinuierlich oder diskontinuierlich durchführen.

Nach beendeter Umsetzung kann das Produkt destillativ abgetrennt werden; flüchtige Jod- bzw. Bromverbindungen werden dabei als Vorlauf abgenommen und gemeinsam mit dem bei Durchführung in der Flüssigphase den Katalysator enthaltenden Destillationssumpf in den Reaktor zurückgeführt. Je nach Art des verwendeten Carbonsäureamids wird dieses mit dem Katalysator oder als eine Destillationsfraktion ebenfalls in den Prozeß zurückgeführt.

Das erfindungsgemäße Verfahren ist jedoch auf eine bestimmte Ausprägung der technischen Ausführung nicht beschränkt.

Die Reaktion erfolgt in Gegenwart von Kohlenmonoxid. Dieses wird zwar gemäß der Stöchiometrie für die Umsetzung nicht benötigt, dient aber zur Stabilisierung des Ameisensäureesters und der aktiven Katalysatorform gegen Zersetzung und nötigenfalls zur Bildung dieser aktiven Katalysatorform aus den eingesetzten Verbindungen. Das Gas kann, gegebenenfalls unter Abzweigen eines kleinen Abfallstroms zum Ausschleusen gasförmiger Verunreinigungen, im Kreis geführt werden. Verunreinigungen des Kohlenmonoxids durch Wasserstoff, Stickstoff, Methan, Kohlendioxid oder andere inerte Gase stören auch in größerer Menge nicht; das Verfahren kann sogar unter Synthesegasdruck durchgeführt werden. Fremdgase sollten aber gering gehalten werden, um zur Einstellung des notwendigen CO-Partialdrucks keinen unerwünscht hohen Reaktionsdruck zu benötigen.

Bei Verwendung von reinem Kohlenmonoxid wird das Verfahren bei Reaktionsdrücken von 2 bis 250 bar, bevorzugt bei 10 bis 100 bar, durchgeführt. Typische CO-Kaltdrücke im bevorzugten Bereich bei einer Durchführung der Reaktion in der Flüssigphase sind 5 bis 80 bar. Der zur Stabilisierung des Eduktes oder des Katalysators vorzulegende Mindestdruck an Kohlenmonoxid ist von der Reaktionstemperatur abhängig und kann bei Bedarf schnell und einfach ermittelt werden.

Die Umsetzungstemperatur des Verfahrens beträgt 140 bis 300 °C, bevorzugt 160 bis 220 °C. Die Reaktionsdauer kann in Abhängigkeit von den übrigen Verfahrensparametern in weiten Grenzen variieren und wird zweckmäßigerweise so eingerichtet, daß ein praktisch vollständiger Umsatz erreicht wird, im allgemeinen in 0,1 bis 10 Stunden.

Eine Reaktionsdauer von zwei Stunden und weniger läßt sich dabei ohne weiteres erreichen.

Es ist leicht einzusehen, daß der Einsatz einer möglichst geringen Menge an Katalysator, vor allem des Rhodiums als dem kostspieligsten Bestandteil, wirtschaftlich wünschenswert ist. Daher ist es von Vorteil, daß das Verfahren im Vergleich zu Katalysatorsystemen, die auf anderen Metallen aufbauen, mit sehr geringem Katalysatoreinsatz auskommt. Geeignet sind Mengen an Rhodium (verbindung), die 0,05 bis 5 mg-atom Metall pro mol Ameisensäuremethylester, bevorzugt 0,2 bis 2 mg-atom/mol, entsprechen. Dabei beträgt das Atomverhältnis Rhodium zu Halogen, bevorzugt Rh/J, 1 : 1 000 bis 1 : 1, bevorzugt 1 : 100 bis 1 : 5, und das Verhältnis Rhodium zu Stickstoffverbindung, berechnet als Atomverhältnis Rh/N, 1 : 100 bis 1 : 1, bevorzugt 1 : 40 bis 1 : 2, wobei das Verhältnis organische Stickstoffverbindung zu Ameisensäuremethylester niedriger als 0,2 mol/mol ist.

Dabei wird ein Verhältnis des J oder Br zu N von größer als 1 eingehalten, um besonders gute Ergebnisse zu erzielen. Verwendet man Verbindungen der VI. Nebengruppe als zusätzliche Komponente, so beträgt das Atomverhältnis zu Rhodium, bevorzugt Rh/Cr, 1 : 100 bis 10 : 1, vorzugsweise 1 : 20 bis 2 : 1.

Es können zwar grundsätzlich auch größere Mengen jeder Komponente eingesetzt werden, jedoch bewirkt das keine Verbesserung und ist wirtschaftlich ungünstig. Bei kleineren Mengen dagegen ist es notwendig, in unerwünschter Weise die Umsetzungsdauer zu verlängern oder die Reaktionsbedingungen zu verschärfen.

Das erfindungsgemäße Verfahren ermöglicht es, Essigsäure in hoher Reinheit durch katalytische

Umlagerung von Ameisensäuremethylester bei hoher spezifischer Katalysatorleistung praktisch quantitativ als alleiniges Produkt zu erhalten. Im folgenden wird das Verfahren durch Beispiele näher erläutert.

Beispiel 1

In einem 100 ml-Autoklaven aus Hastelloy C werden 41 g (683 mmol) Methylformiat, 0,2 g (0,76 mmol) Rhodiumtrichloridtrihydrat 5 g (35,2 mmol) Methyljodid und 1,48 g (20,2 mmol) N,N-Dimethylformamid vorgelegt. Der Autoklav wird druckfest verschlossen und mit Kohlenmonoxid gespült. Anschließend wird Kohlenmonoxid bis 35 bar aufgepreßt, auf 180 °C aufgeheizt, der Reaktionsdruck mittels geringer CO-Zugabe auf 50 bar eingestellt und die Umsetzung unter intensivem Rühren 1,5 Stunden lang durchgeführt. Danach erfolgt schnelles Abkühlen (15 min) durch Einblasen von Preßluft in die Heizung und Entspannen des Autoklaven über einen Abgaswäscher. Nach Zusatz definierter Mengen 1.4-Dioxan als innerem Standard werden der flüssige Reaktionsaustrag und der Wäscherinhalt gaschromatographisch analysiert; es zeigt sich, daß das Methylformiat zur gewünschten Essigsäure umgesetzt ist (Umsatz: 64,4 %). Die Geschwindigkeit der Umsetzung ist hoch; die spezifische Katalysatoraktivität beträgt 205 g Essigsäure/g Rh · h. Man erhält die Essigsäure in einer Selektivität von 91 mol-%.

Das Reaktionsgemisch enthielt neben freier Essigsäure fast ausschließlich noch die dem Methylformiat-Umsatz entsprechenden Mengen Methylacetat und Ameisensäure ungefähr in äquimolarem Verhältnis. Nebenprodukte wie Wasser, CO, Methanol, $H_2$, $CH_4$ oder $CO_2$ waren nicht oder nur in geringer Menge vorhanden. Die Umsetzung zu Essigsäure läßt sich durch bloße Verlängerung der Reaktionsdauer fast quantitativ gestalten.

Beispiel 2

Beispiel 1 wird mit 1,76 g (20,2 mmol) N,N-Dimethylacetamid wiederholt. Der Umsatz an Methylformiat beträgt 54,4 %, die Selektivität zu Essigsäure 88,1 mol-% und die Katalysatoraktivität 168 g Essigsäure/g Rh · h.

Vergleichsbeispiel

Die Reaktion wird wie in Beispiel 1, jedoch ohne N,N-Dimethylformamid und zwei Stunden lang durchgeführt. Der Umsatz beträgt in diesem Fall nur 27 %; die Selektivität für Essigsäure ist 58, die für Methylacetat 22 mol-%. Daneben enthält das Reaktionsgemisch eine dem Methylacetat äquivalente, d. h. gleiche molare Menge Ameisensäure, geringe Mengen (je 1 bis 2 mmol) Methanol und Acetaldehyd und 11 mmol Wasser. Die Katalysatoraktivität ist mit 41 g freier Essigsäure/g Rh · h gering.

**Patentansprüche**

1. Verfahren zur Herstellung von Essigsäure durch katalytische Umlagerung von Ameisensäuremethylester in Gegenwart eines Metallkatalysatorsystems, das Rhodium bzw. eine Rhodiumverbindung, ein Halogen oder eine Halogenverbindung als Promotor und eine organische Stickstoffverbindung enthält, und von Kohlenmonoxid bei erhöhter Temperatur und erhöhtem Druck, dadurch gekennzeichnet, daß man die Umsetzung in der Flüssigphase an einem Metallkatalysatorsystem aus Rhodium bzw. Rhodiumsalzen oder Rhodiumkomplexen, einem Halogen oder einer Halogenverbindung als Promotor und Liganden aus der Gruppe der Carbonsäureamide bei Temperaturen von 140 bis 300 °C und CO-Partialdrücken bei diesen Reaktionstemperaturen von 2 bis 250 bar durchführt, wobei man das Rhodium oder die Rhodiumverbindung in Mengen von 0,05 bis 5 mg-atom Rhodium pro mol eingesetztes Methylformiat einsetzt und ein Atomverhältnis Rhodium : Halogen von 1 : 1 000 bis 1 : 1 und ein Atomverhältnis Rh : N von 1 : 100 bis 1 : 1 einhält, wobei das Verhältnis organische Stickstoffverbindung : Ameisensäuremethylester niedriger als 0,2 mol/mol ist.

2. Verfahren nach Anspruch 1, dadurch gekennzeichnet, daß man die Umsetzung an einem Metallkatalysatorsystem durchführt, das zusätzlich ein Salz oder einen Komplex eines Elementes der VI. Nebengruppe enthält, und ein Atomverhältnis Rhodium : Metall der VI. Nebengruppe von 1 : 100 bis 10 : 1 einhält.

3. Verfahren nach Anspruch 2, dadurch gekennzeichnet, daß man als Metallverbindung der VI. Nebengruppe ein Metallcarbonyl und/oder ein Metallhalogenid einsetzt.

4. Verfahren nach Anspruch 3, dadurch gekennzeichnet, daß man ein Chromcarbonyl und/oder Chromhalogenid einsetzt.

5. Verfahren nach den Ansprüchen 1 bis 4, dadurch gekennzeichnet, daß man als Halogenpromotoren Jod, Brom oder Verbindungen von Jod und/oder Brom einsetzt.

6. Verfahren nach Anspruch 5, dadurch gekennzeichnet, daß man als Halogenpromotor Methyljodid einsetzt.

7. Verfahren nach den Ansprüchen 1 bis 6, dadurch gekennzeichnet, daß man das Rhodium bzw. die

Rhodiumverbindung in Mengen von 0,2 bis 2 mg-atom Rhodium pro mol eingesetztes Methylformiat einsetzt.

8. Verfahren nach den Ansprüchen 1 bis 7, dadurch gekennzeichnet, daß man in dem Metallkatalysatorsystem ein Atomverhältnis Rhodium : Halogen von 1 : 100 bis 1 : 5 und Rh : N von 1 : 40 bis 1 : 2 einhält.

9. Verfahren nach den Ansprüchen 1 bis 8, dadurch gekennzeichnet, daß man in dem Metallkatalysatorsystem ein Atomverhältnis Rhodium : Metall der VI. Nebengruppe von 1 : 20 bis 2 : 1 einhält.

10. Verfahren nach den Ansprüchen 1 bis 9, dadurch gekennzeichnet, daß man die Umsetzung bei einer Temperatur von 160 bis 220 °C und Reaktionsdrücken von 10 bis 100 bar durchführt.

## Claims

1. A process for the preparation of acetic acid by catalytic rearrangement of methyl formate, at a raised temperature and increased pressure, in the presence of a metal catalyst system which contains rhodium or a rhodium compound, a halogen or a halogen compound as a promoter and an organic nitrogen compound, and of carbon monoxide, characterized in that the reaction is performed in a liquid phase with a metal catalyst system comprising rhodium or rhodium salts or rhodium complexes, a halogen or a halogen compound as promoter, and ligands from the carboxamide group, at temperatures of 140 to 300 °C and partial pressures of CO at these reaction temperatures of 2 to 250 bars, wherein rhodium or the rhodium compound is used in amounts of 0.05 to 5 mg-atom rhodium per mol of methyl formate added, the ratio of rhodium atoms : halogen atoms is between 1 : 1.000 and 1 : 1, and the ratio of rhodium atoms : N atoms is between 1 : 100 and 1 : 1, and wherein the ratio of organic nitrogen compound : methyl formate is less than 0.2 mol/mol.

2. A process according to Claim 1, characterized in that the conversion is performed with a metal catalyst system which additionally contains a salt or a complex of an element of subgroup VI, and the ratio of rhodium atoms : atoms of the metal of subgroup VI is between 1 : 100 and 10 : 1.

3. A process according to Claim 2, characterized in that a metal carbonyl and/or a metal halide is employed as the compound of the metal of subgroup VI.

4. A process according to Claim 3, characterized in that a chromium carbonyl and/or a chromium halide is employed.

5. A process according to Claims 1 to 4, characterized in that iodine, bromine or compounds of iodine and/or bromine are employed.

6. A process according to Claim 5, characterized in that methyl iodide is employed as halogen promoter.

7. A process according to Claims 1 to 6, characterized in that rhodium or the rhodium compound is employed in amounts which contain between 0.2 and 2 mg-atom rhodium per mol of methyl formate added.

8. A process according to Claims 1 to 7, characterized in that the ratio of rhodium atoms : halogen atoms in the metal catalyst system is between 1 : 100 and 1 : 5, and Rh : N is between 1 : 40 and 1 : 2.

9. A process according to Claims 1 to 8, characterized in that the ratio of rhodium atoms : atoms of the metal of subgroup VI in the metal catalyst system is between 1 : 20 and 2 : 1.

10. A process according to Claims 1 to 9, characterized in that the reaction is performed at a temperature of 160 to 220 °C and a reaction pressure of 10 to 100 bars.

## Revendications

1. Procédé pour la préparation d'acide acétique par transposition catalytique de formiate de méthyle en présence d'un système catalytique à base de métal qui contient du rhodium ou un composé de rhodium, un halogène ou un composé halogéné en tant que promoteur et un composé organique azoté, et de monoxyde de carbone, à haute température et sous une pression élevée, caractérisé en ce que l'on effectue la réaction en phase liquide en présence d'un système catalytique à base de métal, constitué de rhodium ou de sels de rhodium ou de complexes de rhodium, d'un halogène ou d'un composé halogéné en tant que promoteur, et de ligands choisis parmi des carboxamides, à des températures de 140 à 300 °C et sous des pressions partielles de CO de 2 à 250 bars à ces températures de réaction, en utilisant le rhodium ou le composé de rhodium en quantités de 0,05 à 5 atomes-mg de rhodium par mole de formiate de méthyle utilisé et en maintenant un rapport atomique rhodium : halogène de 1 : 1 000 à 1 : 1 et un rapport atomique Rh : N de 1 : 100 à 1 : 1, le rapport composé organique azoté : formiate de méthyle étant inférieur à 0,2 mole/mole.

2. Procédé selon la revendication 1, caractérisé en ce que l'on effectue la réaction en présence d'un système catalytique à base de métal qui contient en outre un sel ou un complexe d'un élément du groupe VI et on maintient un rapport atomique rhodium : métal du groupe VI allant de 1 : 100 à 10 : 1.

3. Procédé selon la revendication 2, caractérisé en ce que, en tant que composé métallique du groupe VI, on utilise un carbonyle métallique et/ou un halogénure métallique.

4. Procédé selon la revendication 3, caractérisé en ce que l'on utilise un carbonyle de chrome ou un halogénure de chrome.

5. Procédé selon l'une quelconque des revendications 1 à 4, caractérisé en ce que, en tant que promoteur halogéné, on utilise l'iode, le brome ou des composés d'iode et/ou de brome.

6. Procédé selon la revendication 5, caractérisé en ce que, en tant que promoteur halogéné, on utilise l'iodure de méthyle.

7. Procédé selon l'une quelconque des revendications 1 à 6, caractérisé en ce que l'on utilise le rhodium ou le composé de rhodium en quantités de 0,2 à 2 atomes-mg par mole de formiate de méthyle utilisé.

8. Procédé selon l'une quelconque des revendications 1 à 7, caractérisé en ce que l'on maintient dans le système catalytique à base de métal un rapport atomique rhodium : halogène de 1 : 100 à 1 : 5 et un rapport atomique Rh : N de 1 : 40 à 1 : 2.

9. Procédé selon l'une quelconque des revendications 1 à 8, caractérisé en ce que l'on maintient dans le système catalytique à base de métal un rapport atomique rhodium : métal du groupe VI allant de 1 : 20 à 2 : 1.

10. Procédé selon l'une quelconque des revendications 1 à 9, caractérisé en ce que l'on effectue la réaction à une température de 160 à 220 °C et sous des pressions de réaction de 10 à 100 bars.